# FASCICULE DE BREVET EUROPEEN

(11) **EP 4 319 679 B1**
(45) Date de publication et mention de la délivrance du brevet: **08.10.2025**
(21) Numéro de dépôt: 22717014.9
(22) Date de dépôt: 07.04.2022
(51) Int. Cl.: A61C 5/90, A61B 1/24, A61B 90/16

(54) **BLOC À MORDRE ET CALE-BOUCHE COMPRENANT DEUX BLOCS À MORDRE**
BEISSBLOCK UND MUNDPROPST MIT ZWEI BEISSBLOECKEN
BITE BLOCK AND MOUTH WEDGE COMPRISING TWO BITE BLOCKS

(30) Priorité: 08.04.2021 FR 2103620
(43) Date de publication de la demande: 14.02.2024
(73) Titulaire: Chilles, Jean Gabriel, 68420 Gueberschwihr (FR)
(72) Inventeur: Chilles, Jean Gabriel, 68420 Gueberschwihr (FR)
(74) Mandataire: Hautier IP
(86) Numéro de dépôt international: PCT/IB2022/053299
(87) Numéro de publication internationale: WO 2022/215042

(56) Documents cités:
- EP-B1- 2 175 768
- WO-A1-2016/079387
- WO-A2-03/037205
- CN-U- 208 942 234
- US-A- 2 800 896
- US-A- 5 466 153
- US-A- 5 735 691
- US-A1- 2010 291 503
- US-A1- 2012 088 205
- US-A1- 2013 104 912
- US-A1- 2019 099 236

## Description

### Domaine technique

L'invention concerne les domaines des soins orthodontiques et des soins dentaires, et plus particulièrement dans ces domaines, un bloc à mordre et un cale-bouche. De tels dispositifs sont aujourd'hui communément utilisés pour maintenir écartées les mâchoires d'un patient pendant la durée des soins. Ils apportent un confort pour le patient, qui doit fournir un effort musculaire et articulaire soutenu pour maintenir la bouche ouverte pendant la durée des soins. Ils facilitent également le travail du praticien qui doit accéder à certaines zones difficilement accessibles pour réaliser les soins.

Le document US5466153 décrit un bloc à mordre comprenant deux surfaces d'appui opposées configurées de sorte que des dents d'une des mâchoires viennent en appui sur une première surface et des dents de l'autre des mâchoires viennent en appui sur une deuxième surface. Les deux surfaces d'appui sont sensiblement planes et présentent des picots ayant un effet antidérapant. Les deux surfaces planes sont inclinées l'une par rapport à l'autre de sorte que, lorsque le bloc à mordre est positionné à l'intérieur de la bouche d'un patient, des plans dans lesquels s'étendent les surfaces d'appui se coupent selon une droite située approximativement au fond de la bouche du patient. L'expérience montre toutefois qu'un tel bloc est souvent instable. En effet, selon sa morphologie, son amplitude d'ouverture de bouche et l'angulation existante entre sa mâchoire supérieure et sa mâchoire inférieure, un patient peut avoir du mal à appuyer simultanément sur l'ensemble des deux surfaces d'appui. Les picots antidérapants ont peu d'effet dans ces conditions et le bloc s'avère dans la pratique instable. Cette instabilité peut conduire à un déplacement du bloc à mordre vers l'avant ou vers l'arrière de la cavité buccale, ou à un basculement du bloc à mordre dans la cavité buccale.

US5735691A divulgue un bloc à mordre comprenant une surface plane et une surface bombée opposée. Les deux surfaces opposées ne comprennent pas de crans transversaux faisant saillie.

Les documents US2800896A, CN208942234U, US2019099236A1 divulguent d'autres blocs à mordre comprenant au moins une surface d'appui bombée. Ces blocs à mordre ne tiennent cependant pas compte de la présence de cupsides irrégulières sur la surface des dents. Un objet de l'invention est d'améliorer l'appui des dents sur le bloc à mordre, en tenant compte des cuspides des dents.

### Description de l'invention

L'invention est définie par les revendications annexées. L'invention propose un nouveau bloc à mordre adapté pour maintenir écartées les mâchoires d'un patient, bloc comprenant une première surface d'appui et une deuxième surface d'appui, bloc dans lequel les deux surfaces d'appui sont opposées et configurées de sorte que des dents d'une des mâchoires viennent en appui sur la première surface plane et des dents de l'autre des mâchoires viennent en appui sur la deuxième surface, bloc dans lequel la première surfaces est plane.

Le bloc selon l'invention est caractérisé en ce que la deuxième surface d'appui est bombée. Le bloc est ainsi adapté à toutes les morphologies, les amplitudes d'ouverture de bouche et les angulations existantes entre la mâchoire supérieure et la mâchoire inférieure qui varient d'un patient à l'autre. Certaines personnes vont ainsi mordre sur la partie postérieure de la surface bombée, d'autres plutôt sur le dessus de la surface bombée. La stabilité du bloc est ainsi améliorée.

L'invention concerne également un cale-bouche comprenant deux blocs latéraux tels que décrits ci-dessus, blocs latéraux reliés par une partie centrale. La combinaison de deux blocs latéraux apporte un appui des deux côtés de la bouche et limite les tendances au basculement du bloc vers l'intérieure de la bouche. Le cale-bouche apporte ainsi une stabilité et un confort supplémentaires pour le patient.

Selon un mode de réalisation, le cale-bouche comprend également un miroir positionné contre une face avant de la partie centrale, pour améliorer la vision du praticien.

### Brève description des figures

L'invention sera mieux comprise, et d'autres caractéristiques et avantages de l'invention apparaîtront à la lumière de la description qui suit d'exemples de mises en oeuvre de l'invention. Ces exemples sont donnés à titre non limitatif. La description est à lire en relation avec les dessins annexés dans lesquels :
- la figure 1 montre un exemple de réalisation d'un bloc à mordre selon l'invention,
- la figure 2 montre un autre exemple d'un bloc selon l'invention
- la figure 3 montre un exemple d'un cale-bouche selon l'invention
- la figure 4 montre un autre exemple d'un cale-bouche selon l'invention

Dans l'ensemble de la description de la présente demande de brevet, les notions et expressions suivantes sont utilisées :
- les notions "avant" et "arrière" sont définies par rapport à la vision d'un praticien regardant un bloc à mordre ou un cale-bouche en position d'utilisation dans la bouche d'un patient,
- par plan "longitudinal", on entend un plan vertical, s'étendant sensiblement dans une longueur de la bouche d'un patient (plan défini par les directions Y et Z du repère orthonormé représenté sur les figures),
- par axe ou direction "transversal(e)", on entend un axe ou une direction s'étendant sensiblement dans une largeur de la bouche (axe X du repère orthonormé).

### Description détaillée de modes de réalisation de l'invention

Comme dit précédemment, l'invention concerne un bloc à mordre adapté pour maintenir écartées les mâchoires d'un patient. Le bloc comprend une première surface 1 d'appui et une deuxième surface 2 d'appui ; les deux surfaces d'appui sont opposées et configurées de sorte que des dents d'une des mâchoires viennent en appui sur la première surface plane et des dents de l'autre des mâchoires viennent en appui sur la deuxième surface ; la première surface du bloc est plane et, selon l'invention, la deuxième surface 2 est bombée. La première surface d'appui, plane, est le plan de référence pour le positionnement et le calage du bloc dans la bouche du patient. La deuxième surface d'appui, bombée et opposée à la première surface plane rend le bloc adapté à toute forme de mâchoires.

Le bloc est réalisé dans un matériau adapté pour résister à la pression exercée par les mâchoires. Il peut être réalisé être réalisé en plastique dur, ou bien en plastique mou (type silicone par exemple). Il peut être réalisé par injection plastique ou impression 3D.

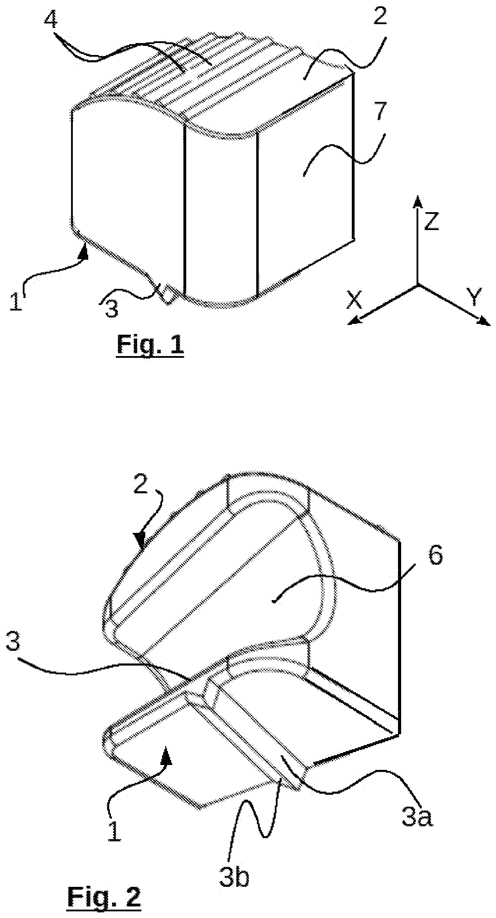

Un unique cran 3 transversal s'étend depuis la surface plane (fig. 1 -2). L'expérience montre qu'un seul cran est préférable pour s'adapter au mieux à la surface supérieure des dents d'une mâchoires. La surface supérieure des dents est en effet très irrégulière, pour chaque personne, du fait de la présence, sur cette surface supérieure, d'une pluralités de cuspides (ou protubérances) irrégulières dans leur forme et leur volume. L'unique cran vient se positionner mécaniquement entre des cuspides, et l'absence de crans supplémentaires sur la surface d'appui plane permet un appui efficace sur la surface plane par plusieurs pointes des cuspides situées autour du cran.

Le cran 3 peut être dessiné (fig. 2) de manière à épouser au mieux la forme des cuspides des dents. Il est doublement asymétrique avec une angulation pour être perpendiculaire à la ligne d'arcade de la mâchoire. Le cran comprend une pente antérieure 3a plus marquée que la pente postérieure 3b de manière à s'opposer à l'effet de pression vers l'avant que la mandibule (mâchoire inférieure) a naturellement en fermant la bouche et en serrant les dents sur un objet dur.

Egalement, une pluralité de nervures transversales 4 peuvent s'étendre sur la surface bombée. La surface d'appui 2 étant bombée, une seule nervure 4 vient se positionner entre des cuspides pour un effet anti-dérapant.

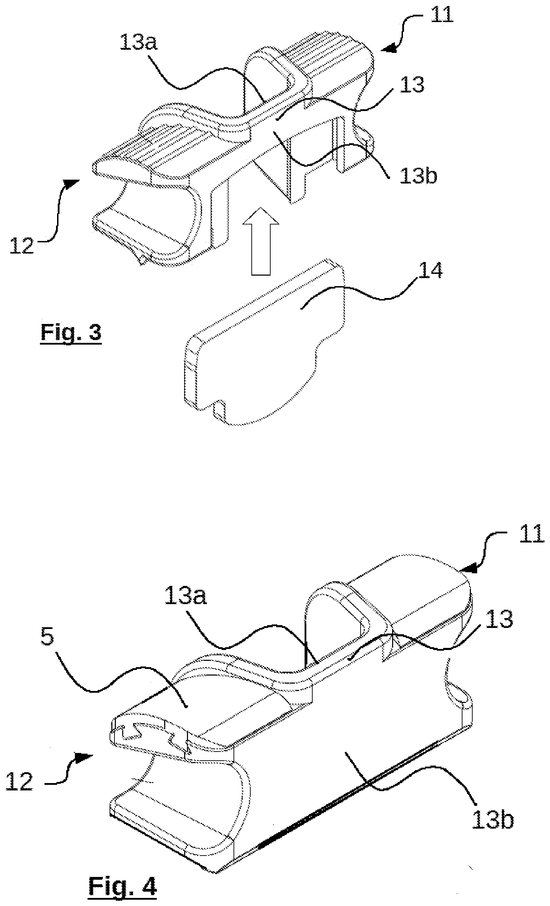

Dans l'exemple des figures 1-2, la surface d'appui bombée 2 est une surface extérieure du bloc. Dans une variante, fig. 3, la surface d'appui bombée 2 est une surface extérieure d'un coussinet 5 fixé, de préférence de manière amovible, sur la surface extérieure du bloc. Le coussinet est réalisé dans un matériau plastique souple, d'une part pour améliorer le confort de l'appui, et d'autre part pour mieux épouser la surface supérieure irrégulière des dents et ainsi augmenter la surface d'appui réelle des dents sur la surface d'appui bombée.

L'utilisation d'un coussinet est notamment intéressante si le bloc lui-même est réalisé en matériau dur. Le coussinet peut être fixé sur la surface extérieure du bloc par exemple par collage ou soudage. Le coussinet peut également être réalisé simultanément avec l'ensemble du bloc par un procédé d'injection bimatière ou d'impression 3D bimatière. Le bloc obtenu est ainsi plus facile à nettoyer et à désinfecter. Pour faciliter le nettoyage également, le coussinet peut encore être fixé de manière amovible sur le bloc. Par exemple sur la fig. 4, le coussinet est fixé au bloc par un assemblage de type queue d'aronde.

Le bloc selon l'invention peut encore comprendre un miroir positionné contre ou dans le prolongement d'une face avant 7 du bloc. Le miroir est configuré de préférence pour former un angle compris dans une plage de de 70 à 110° avec un plan passant par la surface plane 1 du bloc. Le miroir améliore la vision du praticien, par réflexion de la lumière et par vision indirecte lors d'intervention notamment sur la face arrière des dents de devant, incisives et canines notamment. En effet, le miroir reflétant la lumière permet d'éclairer ces zones sombres de la bouche, sans apporter de lumière par un autre instrument susceptible de restreindre l'espace de travail du praticien (amélioration de la vision directe). De plus, le miroir permet une vision indirecte de la zone de travail reflétée dans le miroir. Le miroir peut être fixé sur la face avant 7 du bloc, par exemple par collage ou soudage. Le miroir peut aussi être réalisé par un dépôt de matière réfléchissante sur la face avant 7 du bloc. En variante, le miroir peut être monté amovible, solidarisé au bloc par un assemblage comprenant une ou deux rainures formant glissière pour l'insertion et le maintien du miroir. Le miroir peut ainsi être enlevé pour faciliter le nettoyage de l'ensemble.

Le bloc peut encore comprendre un évidement longitudinal s'étendant entre la première surface d'appui 1 et la deuxième surface d'appui 2, évidement destiné à permettre le passage d'un aspirateur à salive.

Bien que les surfaces d'appui ne soient pas symétriques, le bloc à mordre peut être utilisé au choix dans un sens ou dans l'autre : mâchoire inférieure en appui sur la surface plane 2 et mâchoire supérieure en appui sur la surface bombée 3, ou bien l'inverse.

L'invention concerne également un cale-bouche (fig. 3 -4) comprenant deux blocs à mordre latéraux 11, 12

reliés par une partie centrale 13. Les blocs à mordre latéraux 11, 12 sont réalisés tel que décrit ci-dessus. Dans le cale-bouche la partie centrale 13 peut comprendre une face arrière 13a s'étendant entre les deux blocs et définissant avec des faces latérales des blocs à mordre latéraux, un espace concave configuré pour écarter une langue d'un patient.

Le cale-bouche peut également comprendre un miroir 14 positionné contre une face avant 13b de la partie centrale 13, face avant 13b opposée à la face arrière 13a.

Le miroir peut être fixé sur la face avant 13b de la partie centrale 13, par exemple par collage ou soudage. Le miroir peut aussi être réalisé par un dépôt de matière réfléchissante sur la face avant de la partie centrale 13. En variante, dans l'exemple de la fig. 4, le miroir est monté amovible, solidarisé au cale bouche par un assemblage comprenant deux rainures formant glissière pour l'insertion et le maintien du miroir. Le miroir peut ainsi être enlevé pour faciliter le nettoyage de l'ensemble. Le miroir peut également être enlevé pour agrandir l'espace de travail lorsque le miroir n'est pas nécessaire pour la vision du champ opération.

Selon un mode de réalisation, le miroir est fixé sur la partie centrale de sorte à former un angle compris dans une plage de 70 à 110° avec un plan passant par les surfaces planes 1 des blocs à mordre latéraux 11, 12. L'inclinaison du miroir permet de visualiser au mieux les faces arrières des dents de devant du patient.

Le cale-bouche peut être utilisé de manière à ce que les dents de la mâchoire inférieure appuient sur la surface plane 2 ; l'orientation du miroir permet alors d'éclairer et de visualiser la face arrière des dents de devant de la mâchoire inférieure. Inversement, le cale-bouche peut être utilisé de manière à ce que les dents de la mâchoire supérieure appuient sur la surface plane 2; l'orientation du miroir permet alors d'éclairer et de visualiser la face arrière des dents de devant de la mâchoire supérieure. L'inclinaison du miroir par rapport à la surface plane 2 permet ainsi de bénéficier de deux angles de vue complémentaires.

### Nomenclature

- 1: surface d'appui plane
- 2: surface d'appui bombée
- 3: cran
3a face avant du cran
3b face arrière du cran
- 4: nervures
- 5: coussinet
- 6: évidement
- 7: face avant du bloc
- 11, 12: blocs à mordre latéraux
- 13: partie centrale
13a face arrière de la partie centrale
13b face avant de la partie centrale
- 14: miroir

## Revendications

1. Bloc à mordre adapté pour maintenir écartées les mâchoires d'un patient, bloc comprenant une première surface (1) d'appui et une deuxième surface (2) d'appui, bloc dans lequel les deux surfaces d'appui sont opposées et configurées de sorte que des dents d'une des mâchoires viennent en appui sur la première surface et des dents de l'autre des mâchoires viennent en appui sur la deuxième surface, bloc dans lequel la première surface est plane, et la deuxième surface est bombée, ledit bloc comprenant en outre un unique cran (3) transversal, s'étendant en saillie depuis la surface plane.

2. Bloc selon la revendication 1, dans lequel le cran (3) est doublement asymétrique et présente une angulation perpendiculaire à la ligne d'arcade de la mâchoire, ledit cran (3) comprenant une pente antérieure (3a) plus marquée que la pente postérieure (3b).

3. Bloc selon l'une des revendications 1 à 2, comprenant également une pluralité de nervures (4) transversales s'étendant sur la surface bombée.

4. Bloc selon l'une des revendications 1 à 3 dans lequel la surface bombée est une surface extérieure d'un coussinet (5) en matériau souple fixé, de préférence de manière amovible, sur une surface extérieure du bloc.

5. Bloc selon l'une des revendications 1 à 4 comprenant également un miroir (14) positionné contre ou dans le prolongement d'une face avant (7) du bloc.

6. Bloc selon la revendication 5 dans lequel le miroir est configuré pour former un angle compris dans une plage de de 70 à 110° avec un plan passant par la surface plane 1 du bloc.

7. Bloc selon l'une des revendications 1 à 6, comprenant un évidement longitudinal (6) s'étendant entre la première surface d'appui et la deuxième surface d'appui, évidement destiné à permettre le passage d'un aspirateur à salive.

8. Cale-bouche comprenant deux blocs à mordre latéraux selon l'une des revendications précédentes, blocs à mordre latéraux (11, 12) reliés par une partie centrale (13).

9. Cale-bouche selon la revendication 8 dans lequel la partie centrale (13) comprend une face arrière (13a) s'étendant entre les deux blocs à mordre définissant avec des faces latérales des blocs à mordre latéraux, un espace concave configuré pour écarter une langue d'un patient.

10. Cale-bouche selon l'une des revendications 8 à 9 comprenant également un miroir (14) positionné contre une face avant (13b) de la partie centrale, face avant opposée à la face arrière.

## Patentansprüche

1. Beißblock, der geeignet ist, um die Kiefer eines Patienten auseinanderzuhalten, wobei der Block eine erste Auflagefläche (1) und eine zweite Auflagefläche (2) umfasst, wobei in dem Block die beiden Auflageflächen gegenüberliegen und so eingerichtet sind, dass Zähne eines der Kiefer auf der ersten Fläche aufliegen und Zähne des anderen der Kiefer auf der zweiten Fläche aufliegen, wobei im Block die erste Fläche eben ist und die zweite Fläche gewölbt ist, wobei der Block ferner eine einzige Querraste (3) umfasst, die sich von der ebenen Oberfläche heraus erstreckt.

2. Block nach Anspruch 1, wobei die Raste (3) doppelt asymmetrisch ist und eine senkrechte Abwinkelung zur Kieferbogenlinie des Kiefers aufweist, wobei die Raste (3) eine vordere Neigung (3a) aufweist, die steiler ist als die hintere Neigung (3b).

3. Block nach einem der Ansprüche 1 bis 2, der auch eine Vielzahl von Querrippen (4) umfasst, die sich über die gewölbte Oberfläche erstrecken.

4. Block nach einem der Ansprüche 1 bis 3, wobei die gewölbte Oberfläche eine Außenfläche eines Polsters (5) aus weichem Material ist, das vorzugsweise abnehmbar an einer Außenfläche des Blocks befestigt ist.

5. Block nach einem der Ansprüche 1 bis 4, der auch einen Spiegel (14) umfasst, der an oder in Verlängerung einer Vorderseite (7) des Blocks positioniert ist.

6. Block nach Anspruch 5, wobei der Spiegel so eingerichtet ist, dass er einen Winkel im Bereich von 70 bis 110° mit einer Ebene bildet, die durch die ebene Oberfläche 1 des Blocks verläuft.

7. Block nach einem der Ansprüche 1 bis 6, umfassend eine Längsaussparung (6), die sich zwischen der ersten Auflagefläche und der zweiten Auflagefläche erstreckt, wobei die Aussparung dazu bestimmt ist, den Durchgang eines Speichelsaugers zu ermöglichen.

8. Mundschutz, der zwei Seitenbeißblöcke nach einem der vorhergehenden Ansprüche umfasst, Seitenbeißblöcke (11, 12), die durch einen Mittelteil (13) verbunden sind.

9. Mundschutz nach Anspruch 8, wobei der Mittelteil (13) eine Rückseite (13a) umfasst, die sich zwischen den beiden Beißblöcken erstreckt und mit Seitenflächen der Seitenbeißblöcke einen konkaven Raum definiert, der so eingerichtet ist, dass er eine Zunge von einem Patienten wegbewegt.

10. Mundschutz nach einem der Ansprüche 8 bis 9, auch umfassend einen Spiegel (14), der gegen eine Vorderseite (13b) des Mittelteils positioniert ist, Vorderseite gegenüber der Rückseite.

## Claims

1. A bite block adapted to hold apart the jaws of a patient, a block comprising a first bearing surface (1) and a second bearing surface (2), a block wherein the two bearing surfaces are opposite and configured so that teeth of one of the jaws abut the first surface and teeth of the other of the jaws abut the second surface, a block wherein the first surface is flat, and the second surface is curved, said block further comprising a single transverse notch (3), extending protruding from the flat surface.

2. Block according to claim 1, wherein the notch (3) is doubly asymmetrical and has an angulation perpendicular to the arch line of the jaw, said notch (3) comprising a steeper anterior slope (3a) than the posterior slope (3b).

3. Block according to one of claims 1 to 2, also comprising a plurality of transverse ribs (4) extending over the curved surface.

4. Block according to one of claims 1 to 3, wherein the curved surface is an outer surface of a cushion (5) made of soft material attached, preferably removably, to an outer surface of the block.

5. Block according to one of claims 1 to 4, also comprising a mirror (14) positioned against or in line with a front face (7) of the block.

6. Block according to claim 5, wherein the mirror is configured to form an angle comprised in a range of from 70 to 110° with a plane passing through the flat surface 1 of the block.

7. Block according to one of claims 1 to 6, comprising a longitudinal recess (6) extending between the first bearing surface and the second bearing surface, a recess intended to allow passage of a saliva aspirator.

8. Mouth wedge comprising two lateral bite blocks according to one of the preceding claims, lateral bite blocks (11, 12) connected by a central portion (13).

9. Mouth wedge according to claim 8, wherein the central portion (13) comprises a rear face (13a) extending between the two bite blocks defining, with lateral faces of the lateral bite blocks, a concave space configured to move away a tongue of a patient.

10. Mouth wedge according to one of claims 8 to 9, also comprising a mirror (14) positioned against a front face (13b) of the central portion, a front face opposite the rear face.
